# EUROPEAN PATENT APPLICATION

(11) **EP 2 572 719 A1**
(43) Date of publication of application: **27.03.2013**
(21) Application number: 11783116.4
(22) Date of filing: 13.05.2011
(51) Int. Cl.: A61K 31/785, A61P 19/00

(54) **USE OF POLOXAMINES AS INDUCERS OF THE OSTEOGENIC DIFFERENTIATION OF MESENCHYMAL CELLS**

(30) Priority: 18.05.2010 ES 201000670
(71) Applicant: Universidade De Santiago De Compostela, 15782 Santiago de Compostela (ES)
(72) Inventor: ALVAREZ LORENZO, Carmen, E-15782 Santiago de Compostela (ES); REY RICO, Ana, E-15782 Santiago de Compostela (ES); SILVA RIVERA, Teresa, E-15782 Santiago de Compostela (ES); COUCEIRO FOLLENTE, José, E-15782 Santiago de Compostela (ES); CONCHEIRO NINE, Angel, E-15782 Santiago de Compostela (ES)
(74) Representative: ABG Patentes, S.L.
(86) International application number: PCT/ES2011/070348
(87) International publication number: WO 2011/144785

(57) **Abstract**

The invention describes for first time the activity of poloxamines as inducers in the differentiation of mesenchymal or progenitor cells. In particular, the invention demonstrates that poloxamines have an osteogenic ability. The invention relates to the use of one or more poloxamines or a pharmaceutical composition comprising same for the preparation of a drug for treting patients with bone defects in general. The invention also relates to a method for inducing the differentiation of mesenchymal or progenitor cells into osteoblasts by bringing said cells into contact with a sterilised aqueous dispersión or solution of one or more poloxamines.

## Description

### FIELD OF THE INVENTION

The present invention relates to the field of the induction of the differentiation of mesenchymal and stem cells into osteoblasts.

### BACKGROUND OF THE INVENTION

Under certain pathological conditions (complex fractures, traumatisms, treatment of bone tumors and replacement of joints or congenital faults) the damaged bone is not spontaneously formed or regenerated. To restore its structural and functional integrity, autografts or allogenic bone from tissue banks can be applied, but this is not exempt from adverse effects that can become serious (Bauer and Muschler, Clin. Orthop. 10-27, 2000). Moreover, the therapy for bone repairment can be supplemented with the biochemical stimulation of the local healing by means of the administration of growth factors that promote osteoblasts formation (Lieberman et al., J. Bone Joint Surg. Am. 84A, 1032-1044, 2002; Finkemeier. J. Bone Joint Surg. Am. 84, 454-464, 2002). The therapeutic use of the growth factors raises important difficulties derived from their short biological lifetime, deficient stability, tisular selectivity and potential toxicity and carcinogenic activity. These facts make the search of synthetic molecules with osteogenic capacity, better safety/efficiency ratio and more economically affordable, a very active research area (Chun-Ya E. Han y col. Bioorg. Med. Chem. Lett. 19, 1442-1445, 2009). In this regards, the osteogenic activity of simvastatin has been already pointed out (Ayukawa y col., J. Oral Rehab. 37, 123-130, 2010).

Patent WO 2007/7108689 covers an ex vivo method that consists in the addition of a polyamine on a culture medium with stem cells in suspension. It is said that the polyamines are the natural molecules spermidine, spermin, and their precursors omitine and putrescine. Putrescine structure is H₂N-(CH₂)₄-NH₂; omitine is the amino acid H₂N-(CH₂)₃-CH(NH₂)-COOH; spermidine is the triamine H₂N-(CH₂)₄-NH-(CH₂)₃-NH₂; and spermine is the tetramine H₂N-(CH₂)₃-NH-(CH₂)₄- NH-(CH₂)₃-NH₂. These four molecules are amines or polyamine of low molecular weight that have amine groups at one or both ends and the distance between the amine groups is of 3 or more carbon atoms. Ming Cai et al. (Pharmazie 63, 751-756, 2008) has described the osteogenic activity of CBMIDA (catechol-3,6-bis(methyleniminodiacetic acid)).

On the other hand, block copolymers that can undergo in situ gelling phenomena have been shown very suitable components for the preparation of syringeable systems of growth factors that can form a depot in the administration site. Poly(ethylene oxide)-poly(propylene oxide)- poly(ethylene oxide), PEO-PPO-PEO, block copolymers of the Pluronic family have been broadly investigated as delivery systems of growth factors, that are able to retain them and to sustain their release in the environment of the critical defect to be repaired (Clokie et al. Plast. Reconstr. Surg. 105, 628-637, 2000). Pluronics by themselves, in the absence of growth factors, do not show osteogenic activity although they can induce the differentiation of mesemchymal stem cells to adipocytes (Vashi et al. Biomaterials 29, 573-579, 2008).

Other family of PEO-PPO block copolymers is that of the poloxamines or Tetronics, which have a start-like structure with four PEO-PPO arms (with hydroxyl ends) joint together through a central ethylenediamine group. Poloxamines are commercially available in a broad range of molecular weights and EO/PO molar ratios (Chiappetta and Sosnik. Eur. J. Pharm. Biopharm. 66, 303-317, 2007). Poloxamines are used as drug solubilizer and stabilizer agents, as stealth components of nanoparticles surfaces, as hydrogel components for controlled release of drugs, and as components of multipurpose liquids for cleaning and storage of contact lenses (Alvarez-Lorenzo et al. Frontiers in Bioscience E2, 424-440, 2010).

Poloxamines have been also proposed as components of growth factors delivery systems for the treatment of critical bone defects. For example, Tae et al. (Composite comprising polysaccharide-functionalized nanoparticle and hydrogel matrix, a drug delivery system and a bone defect replacement matrix for sustained release comprising the same, and the preparation method thereof. U.S. Pat. Appl. Publ. (2007), Cont.-in-part of U.S. Ser. No. 391,480. CODEN: USXXCO US 2007248675 A1) described the preparation and the use of nanoparticles coated with biocompatible emulsifying polymers, among which poloxamines are cited, in order to regulate the release of growth factors. On the other hand, patent WO 94/25080 describes a method that consists in mixing a hydrogel with cells to be implanted in an animal. Poloxamines are cited among the components of the hydrogels. In these systems, poloxamines only act as inert excipients. The possibility of that poloxamines act as actives substances have been never described nor considered.

### DESCRIPTION OF THE INVENTION

The present invention describes, for first time, that the poloxamines themselves induce the differentiation of mesenchymal or stem cells. Particularly, it is shown they have osteogenic capacity.

Therefore, the invention provides methods for the treatment of patients suffering bone defects in general, by the administration of a therapeutically effective amount of a pharmaceutical composition of poloxamines. Poloxamines are the active ingredient in these compositions, thereof, the addition of other active substance is not required. According to one aspect, the invention is directed to the use of one or more poloxamines for the manufacturing of a medicinal product; according to a particular embodiment, the invention is directed to the use of one or more poloxamines for the manufacturing of a medicinal product for inducing the differentiation of mesenchymal or stem cells; in a further particular embodiment, for inducing the differentiation of mesenchymal or stem cells into osteoblasts.

According to a particular embodiment, the invention is directed to the use of one or more poloxamines for the manufacturing of a medicinal product for the treatment of bone defects generated by comminutes fractures.

According to a particular embodiment, the invention is directed to the use of one or more poloxamines for the manufacturing of a medicinal product for the treatment of fractures associated to osteopenia. Among those fractures, the distal radius fractures are typical.

According to a particular embodiment, the invention is directed to the use of one or more poloxamines for the manufacturing of a medicinal product for the treatment of pseudarthrosis.

According to a particular embodiment, the invention is directed to the use of one or more poloxamines for the manufacturing of a medicinal product for the treatment of the loss of bone stock. This loss of the bone may be due to different problems such as the response to several materials with the consequence of osteolysis and/or stress shielding, as occur in the articular prosthesis, in essential bone cysts, intraosseous ganglion and spinal fusion.

According to other embodiment, the invention is directed to one or more poloxamines for use as medicinal product. According to other embodiment, the invention is directed to one or more poloxamines for use in inducing the differentiation of mesenchymal or stem cells; in a further particular embodiment, for inducing the differentiation of mesenchymal or stem cells into osteoblasts. According to other embodiment, the invention is directed to one or more poloxamines for use in the treatment of bone defects generated by comminutes fractures.

According to a particular embodiment, the invention is directed to one or more poloxamines for use in the treatment of fractures associated to osteopenia. Among those fractures, the distal radius fractures are typical.

According to a particular embodiment, the invention is directed to one or more poloxamines for use in the treatment of pseudarthrosis.

According to a particular embodiment, the invention is directed to one or more poloxamines for use in the treatment of the loss of bone stock. This loss of the bone may be due to different problems such as the response to several materials with the consequence of osteolysis and/or stress shielding, as occur in the articular prosthesis, in essential bone cysts, intraosseous ganglion and spinal fusion.

According to an aspect, the invention is directed to a method for inducing the differentiation of mesenchymal or stem cells into osteoblasts comprising administration to a patient in that need thereof a therapeutically effective amount of one or more poloxamines; according to a particular embodiment, the invention is directed to a method for inducing the differentiation of mesenchymal or stem cells into osteoblasts comprising administration to a patient in that need thereof a therapeutically effective amount of one or more poloxamines.

According to a particular embodiment, the invention is directed to a method for treatment of bone defects generated by comminutes fractures, the method comprising administering a therapeutically effective amount of one or more poloxamines to a patient in the need of said treatment.

According to a particular embodiment, the invention is directed to a method for treatment of fractures associated to osteopenia, the method comprising administering a therapeutically effective amount of one or more poloxamines to a patient in the need of said treatment. Among those fractures, the distal radius fractures are typical.

According to a particular embodiment, the invention is directed to a method for treatment of pseudarthrosis, the method comprising administering a therapeutically effective amount of one or more poloxamines to a patient in the need of said treatment. According to a particular embodiment, the invention is directed to a method for treatment of the loss of bone stock, the method comprising administering a therapeutically effective amount of one or more poloxamines to a patient in the need of said treatment. This loss of the bone may be due to different problems such as the response to several materials with the consequence of osteolysis and/or stress shielding, as occur in the articular prosthesis, in essential bone cysts, intraosseous ganglion and spinal fusion.

Poloxamines present advantages versus substances with osteogenic ability previously described:
- Since poloxamines are synthetic polymers, they have a well defined and known structure, and they were included in numerous trials for biomedical applications, and their use is authorized for cosmetics and cleansing products and preservatives for contact lenses;
- Poloxamines are cytocompatible even at high concentrations and therefore they have a wider safety range than that of growth factors;
- It has been found so far that poloxamines can exert any pharmacological activity; thus, it is not foreseeable that the use of poloxamines can lead to relevant collateral effects;
- They are commercialized at much lower prices that the growth factors, and consequently they offer better cost/effectiveness ratio;
- Poloxamines can stand conventional processes of sterilization;
- In the concentrations interval suitable for exerting the osteogenic activity, poloxamines allow the preparation of compositions that behave as syringeable implants; namely, as in situ gelling systems that can be easily applied by means of an injection and that remain in the application site for prolonged time.

An additional advantage of the invention is that, for the use of poloxamines as osteoinductor, a previous modification of their structure is not required, nor the addition of growth factors neither additional osteogenic substances.

According to the present invention, "poloxamines" refers to poly(ethylene oxide) (PEO) and poly(propylene oxide) (PPO) block copolymers which present a star shape that bear four PEO-PPO arms with hydroxyl groups at the end of the chain; these four arms are connected through a central ethylenediamine group, and they are represented by the general formulas I and II: 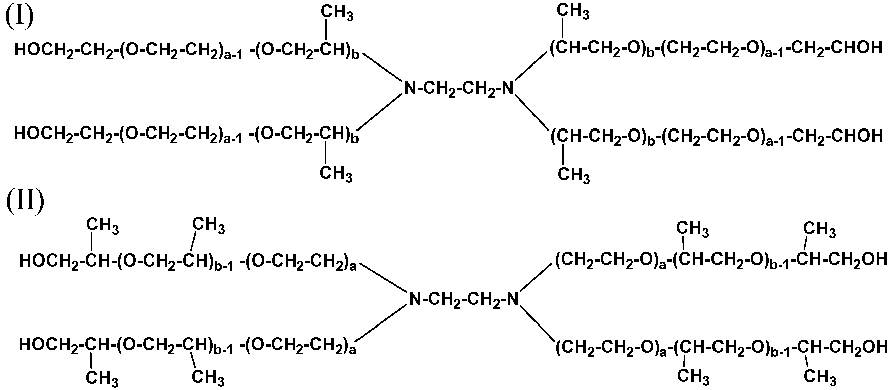 wherein a is from 1 to 150 and b is from 1 to 150.

Poloxamines are commercially available in a wide range of molecular weights and molar relations EO/PO (BASF catalog, The Chemical Company, North American Edition, in the section of brands in the classification of chemicals). Examples of poloxamines useful for the invention are Tetronic (BASF's registered trademark) 304, 701, 901, 904, 908, 1107, 1301, 1304, 1307, 90R4 y 150R1 characterized by the properties specified in table 1.

**Tabla 1.**

| Tetronic | Molecular Weight (Da) | EO units by block (a) | PO units by block (b) | Hydrophilic-lipophilic balance (HLB) | Cloud point 1 % (°C) |
|---|---|---|---|---|---|
| 304 | 1650 | 3.7 | 4.3 | 12-18 | 75 |
| 701 | 3600 | 2.1 | 14.0 | 1-7 | 18 |
| 901 | 4700 | 2.7 | 18.2 | 1-7 | 20 |
| 904 | 6700 | 15 | 17 | 12-18 | 74 |
| 908 | 25000 | 114 | 21 | > 24 | > 100 |
| 1107 | 15000 | 60 | 20 | 18-23 | > 100 |
| 1301 | 6800 | 4 | 26 | 1-7 | 16 |
| 1304 | 10500 | 21.4 | 27.1 | 12-18 | --- |
| 1307 | 18000 | 72 | 23 | > 24 | > 100 |
| 90R4 | 6900 | 16 | 18 | 1-7 | 43 |
| 150R1 | 8000 | 5 | 30 | 1-7 | 20 |

According to other aspect, the invention is directed to a composition pharmaceutically acceptable consisting of one or more poloxamines and one or more excipients. According to a particular embodiment, the composition is suitable for inducing the differentiation of mesenchymal or stem cells. According to a particular embodiment, the composition is suitable for inducing the differentiation of mesenchymal or stem cells into osteoblasts.

According to a preferred embodiment, the composition pharmaceutically acceptable is in the form of solution or aqueous dispersion.

According to another embodiment, the invention is directed to a pharmaceutical composition consisting of a solution or aqueous dispersion of one or more poloxamines for the treatment of bone defects generated by comminutes fractures, fractures associated to osteopenia, pseudarthrosis and loss of bone stock.

According to a particular embodiment, the invention is directed to the use of one or more poloxamines for the manufacturing of said pharmaceutical compositions.

According to the present invention, "composition pharmaceutically acceptable" refers to a composition for a medicinal product; preferred for its parenteral administration. For example, the pharmaceutical compositions of the invention can be administered as an implant by injection in an specific zone wherein the patient is suffering the pathology, which forms a gel at the temperature of the body and produces effects for an extended time in the zone to be treated. Composition pharmaceutically acceptable refers, for example, to a composition which allows its approval by the regulatory agencies as FDA or EMA.

According to the present invention, "pharmaceutically acceptable excipient" refers to any substance in a pharmaceutical composition different from the active ingredient. Said excipients can be liquids, sterile, as for example water and oils, including those of origin in the petrol, animal, vegetable or synthetic, as peanut oil, soy oil, mineral oil, sesame oil, and similar, disintegrate, wetting agents, solubilizing agents, antioxidant, antimicrobial agents, isotonic agents, stabilizing agents or diluents. Suitable adjuvants and /or pharmaceutical carriers are described in "Remington's Pharmaceutical Sciences" by E.W. Martin.

According to other particular embodiment, the invention refers to a viscoelastic gel obtainable by a process comprising contacting a) a sterile aqueous solution or dispersion of one or more poloxamines, with b) mesenchymal or stem cells, being the total concentration of poloxamines in the step a) higher than the gelling critical concentration at the temperature of the medium wherein the mesenchymal or stem cells are. According to the present invention, "gelling critical concentration" refers to that concentration of a solution of poloxamine to which the transition sol-gel undergoes at the temperature of the medium wherein the mesenchymal or stem cells are.

According to a particular embodiment, when the total concentration of poloxamines is higher than the gelling critical concentration at the temperature of the medium wherein the mesenchymal or stem cells are, the composition must be keep between 3°C and 7°C until the application. When this application is carried out by a syringe, keeping cold ensures a good syringeability.

The mesenchymal or stem cells can be *in vivo* or *in vitro.*

According to a particular embodiment, the mesenchymal or stem cells of the step b) are in an *in vitro* culture medium. The process can be carried out by a pipette or a syringe.

According to a preferred embodiment, the total concentration of poloxamines, in the sterile aqueous solution or dispersion of one or more poloxamines, is between 2% and 30%. More preferably, the total concentration of poloxamines is between 10% and 20%. According to a particular embodiment, the invention refers to a process for the manufacturing of a pharmaceutical composition, as described previously, which consists of an sterile aqueous solution or dispersion of one or more poloxamines comprising a) mixing one or more poloxamines in an aqueous media to a pH between 6.5 and 7.5 and at a temperature between 0°C and 37°C, and b) sterilization of the mixture.

According to a particular embodiment, the process comprises an additional step further the step a) and before the step b), which consists of adding an isotonic agent.

The term "isotonic agent" refers to any substance which increases the osmotic pressure of a aqueous solution as for example sodium chloride, glycerin, glucose, mannitol or sorbitol.

The step b) for the sterilization can be carried out according to known process by a skilled person, between them, into the common practice are the following ones: filtration under sterile conditions, moist heat sterilization in the final container, or ionizing or non-ionizing radiations.

According to a particular embodiment, the invention refers to a method for inducing the differentiation of mesenchymal or stem cells into osteoblasts comprising contacting a) a sterile aqueous solution or dispersion of one or more poloxamines, with b) mesenchymal or stem cells. According to a particular embodiment, the mesenchymal or stem cells are in an *in vitro* culture media and the methos is carried out *in vitro.* According to a particular embodiment, the mesenchymal or stem cells do not include mother cells coming from human embryos.

According to a particular embodment, the solution or dispersion of the step a) is administered to animals or human beings in vivo, in particular, by injection in the zone wherein the differentiation of mesenchymal or stem cells into osteoblasts must be induce.

Therefore, when the concentration of total poloxamines is higher than the gelling critical concentration at the temperature of the body, a depot will be formed in situ with a semisolid or viscoelastic gel consistence.

### Description of the figures

Figure 1. Cell viability after 24 h in contact with solutions of poloxamines (n=3) in different concentrations: 0.01% w/w (white columns), 0.1% w/w (clear grey columns), 1% w/w (dark grey columns) and 5% w/w (black columns).
Figure 2. Storage (G', solid symbols) and loss (G", open symbols) moduli of 20% w/w Tetronic solutions in Dulbecco's Modified Eagle Medium (DMEM) before and after being autoclaved.
Figure 3. Alkaline phosphatase (ALP) activity of cells cultured in the presence of various poloxamines. Bioactivity of positive and negative controls are also shown.
Figure 4. Increase in osteogenic marker genes expression induced by poloxamines T908, T1107 and T1307 and poloxamer Pluronic F127, both in the absence and in the presence of rhBMP-2. Osteogenic marker genes expression was observed using RT-PCR at various days. The expression of GAPDH was used to normalize gene expression levels.

### Examples

The following examples are illustrative of the invention and should not be considered as limitants of the same. Examples are provided to illustrate the cytocompatibility, the gelling ability and osteoinductive capability ofpoloxamines in solution.

### General method

A poloxamine or combinations of various poloxamines were dissolved under stirring in water or buffer medium of adequate pH (in the 6.5 to 7.5 range), preferably at pH 7.4. Total concentration in poloxamine can be between 2 to 30%, preferably between 10 to 20%. The temperature of the solution can be between 0 and 37°C, preferably between 4 and 20°C in order to avoid gel formation during the preparation of the solution. The osmotic pressure of the solution can be adjusted with NaCl.

The so lution of po loxamine is sterilized by wet heating (autoclave) at 121°C for 20 min.

### Example 1. Cytocompatibility of poloxamines

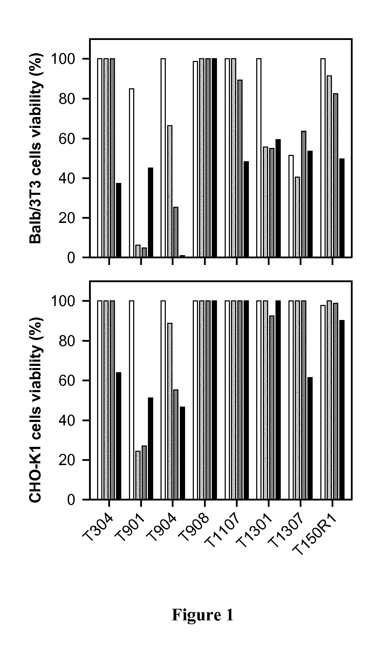

Tetronic 304, 901, 904, 908, 1107, 1301, 1307 and 150R1 solutions (10% w/w) were prepared by dissolving the required amount of each polymer in phosphate buffer pH 7.4. The solutions were filtered by 0.2 µm membranes (MillexTM, Millipore, USA). Balb/3T3 and CHO-K1 cells were cultured in D-MEM without phenol red, containing fetal bovine serum (10% w/v) and gentamicine (130 µg/ 100 ml). 5000 cells/well (50 µl medium) were seeded in 96-well plates and 50 µl of poloxamine solutions (conveniently diluted to 0.01, 0.1, 1 and 5% w/v) were added. Plates were incubated for 24 hours at 37 °C, in a 5% CO2 humidified atmosphere. Cells survival was evaluated at 24 hours by using the Cytotoxicity Detection Kit^{PLUS} (LDH) and the absorbance was measured at 490 nm. Figure 1 shows the results obtained. Tetronic 908, 1107, 1301 and 1307, with large molecular weight and hydrophilic character, showed high cytocompatibility.

### Example 2. Osteoblasts viability

Tetronic 908, 1107, 1301 and 1307 solutions at 20% w/w were prepared in D-MEM without phenol red at 4°C and filtered through 0.2 µm membranes. SAOS-2 cells were cultured in D-MEM supplemented with 10% w/v fetal bovine serum and gentamicine (130 µg/ 100 ml). 200,000 cells/well (1.5 ml) were seeded in 24-well plate and 0.5 ml poloxamine solution was added. Plates were incubated for 24 hours at 37 °C, in a 5% CO2 humidified atmosphere. Then, the cells were detached by addition of tripsine and incubated at 37°C for 5 min. After incubation, cells were centrifuged (1400 rpm for 4 min) and resuspended in fresh D-MEM. Aliquots (100 µL) of cell suspension diluted with phosphate buffer pH 7.4 (200 µL) were transferred to a cytospin and centrifuged in order to fix cells on a microscope slide. Finally, a calcein/propidium iodide staining was done to assess live/dead populations. Viability values were above 90% after 24 h in contact with the 20% poloxamine.

### Example 3. Ability of poloxamines to form gels at body temperature

The storage or elastic (G') and the loss or viscous (G") moduli of 20% w/w Tetronic 908, 1107, 1301 and 1307 solutions in phosphate buffer pH 7.4 and D-MEM were evaluated. To determine the influence of temperature on both moduli, tests were carried out at 5 rad/s from 15°C to 45°C with a heating rate of 2°C/min. Test were carried out with aliquots sterilized (autoclave) at 121°C for 20 min and others avoiding the sterilization.

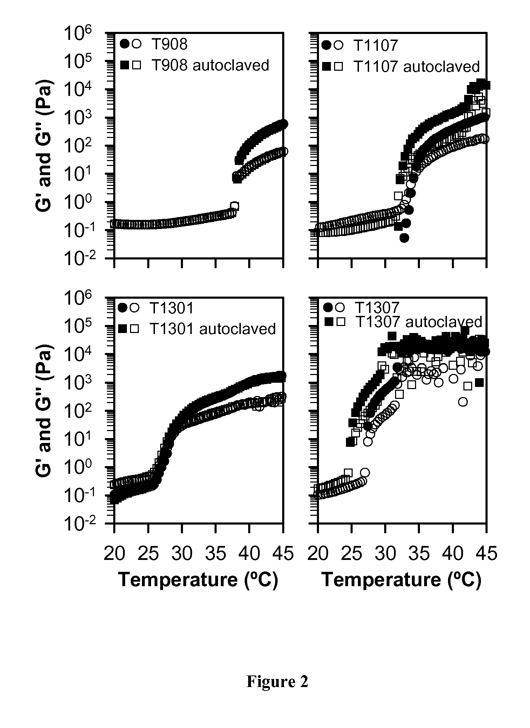

At 20°C the solutions showed low viscosity and were easy to deliver through a syringe. The gel temperature of Tetronic 908 was 33°C and that of Tetronic 1107, 1301 and 1307 was 25°C (Figure 2). At 37°C all systems behaved as gels with storage modulus in the 10³ - 10⁴ Pa. Autoclaving did not alter the alter the rheological behavior of the poloxamine solutions.

### Example 4. Osteoinductive capability

Mesenchymal stem cells were cultured in MesenPRO RSTM medium supplemented and seeded (3•10⁴ cells/well, 1.5 ml) in 6-well plates. 200 µL of solution ofpoloxamine (20% w/w in phosphate buffer at pH 7.4, equivalent to 40 mg of poloxamine) were placed into the upper compartment of 6-well plate Transwell® permeable supports. Negative and positive controls were carried out with cells in culture medium (neither copolymer nor rhBMP-2 added) and cells in osteogenic differentiation medium (10 mM β-glycerophosphate, 100 nM dexametasone and 50 µM ascorbic acid in the culture medium), respectively. Plates were incubated at 37 °C, in a humidified atmosphere with 5%CO₂. The medium was replaced twice a week.

To carry out the alkaline phosphatase (ALP) assay, cells were lysed at 3, 7, 14 and 23 days by addition of 150 µl Tris HCl buffer 10 mM pH 7.5 with 0.1% Triton X-100. Samples were exposed to 3 freezing (-80°C)/unfreezing cycles (45 min per cycle). Lysates were cleared by centrifugation at 14,000 rpm for 15 minutes at 4°C. The samples (50 µl) were incubated with ALP substrate (150 µl) in 96-well plates at 37°C for 30 min. The absorbance was read at 405 nm using an ELISA plate reader. A p-nitrophenylphosphate calibration curve was used to determine ALP concentration. Each ALP activity measurement was normalized by the protein content, which was measured by the BCA protein assay. Results were reported as nanomoles ALP/min/mg protein. Various hystochemical analyses were carried out in triplicate. ALP activity was visualized at 14 days as follows. The cells were fixed using 4% paraformaldehide solution for 5 min, washed with phosphate buffer, incubated at darkness with 0.1% naphthol ASMX-phosphate and 0.1% violeta fast in 56 mM AMPD (2-amino-2-methyl propanediol) and observed at optic microscopy. Alizarin red staining was used to detect the mineralization of the matrix at 14 and 23 days. Cells were fixed with cold ethanol:water 70:30 v/v for 1 hour, washed with water twice, stained with 2% alizarin red for 30 minutes, washed with water three times and observed to the microscopy.

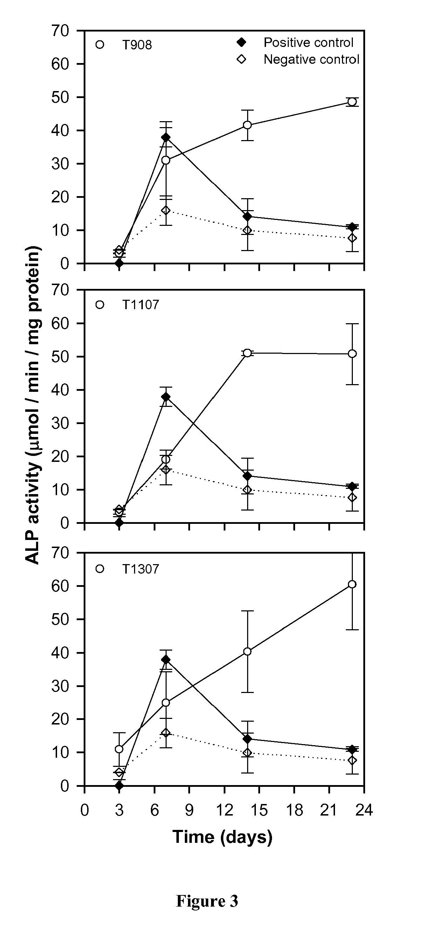

Figure 3 shows the ALP activity profiles obtained for poloxamines Tetronic 908, 1107 and 1307 y the negative and the positive controls of osteogenicity Negative controls revealed that the ALP activity of mesenchymal cells slightly increased at day 7, returning then to basal values. The ALP activity of the cells cultured in the osteogenic medium (positive control) showed a maximum at day 7, which was much more intense than that observed for the negative control. Poloxamine gels by themselves induced a progressive increase in ALP activity for 2 to 3 weeks. Poloxamine gels led to the proliferation of mesemchymal stem cells during the first week and then caused the differentiation to osteoblasts in the 2 to 3 week. Alizarin red staining revealed mineralized nodules in the cell cultures containing Tetronic 908, 1107 and 1307 at day 23.

### Example 5. Reverse transcription-polymerase chain reaction (RT-PCR) analysis

Mesenchymal stem cells were cultured in MesenPRO RSTM medium (Gibco, Invitrogen, Spain) supplemented with 1% glutamine and 1% antimicrobial agents (penicillin, streptomycin, and amphotericin B) and seeded (3•10⁴ cells/well, 2.5 ml) in 6-well plates. 200 µL of poloxamine dispersion (20% w/w), with and without rh-BMP2, were placed into the upper compartment of 6-well plate Transwell® permeable supports. Thus, the amounts of copolymer (poloxamine or poloxamer) and rhBMP-2 added to each well were 40 mg and 7.4 µg, respectively. Negative and positive controls were carried out with cells in culture medium (neither copolymer nor rhBMP-2 added) and cells in osteogenic differentiation medium (10 mM β-glycerophosphate, 100 nM dexametasone and 50 µM ascorbic acid in the culture medium). Plates were incubated at 37 °C, in a humidified atmosphere with 5% CO₂. The medium was replaced twice a week. Cells were observed at inverted microscopy (10x) to discard possible contamination by bacterias. Each experiment was carried out in triplicate and repeated twice. Total cellular RNA was extracted at days 7, 10 and 14 using TRIzol® (Invitrogen, Spain) according to the manufacturer's protocol. Phase separation was carried out using chloroform and the resultant RNA pellets were washed with ethanol:water 70:30 v/v, dried under air and resuspended in DEPC-treated water. The absorbance of RNA systems was measured at 260 nm using a Biophotometer (Eppendorf, Germany) and RNA concentration was calculated. RNA (5µg) was reversed transcripted into cDNA using Maloney murine leukemia virus (M-MLV) reverse transcriptase and random hexamers (Invitrogen, Spain). Reverse transcription (RT) conditions were 37°C for 50 min, 42°C for 15 min and 95°C for 5 min. To assess mRNA expression, a semiquantitative RT-PCR method was used by measuring PCR products during the exponential phase of cDNA amplification. The generated cDNA was amplified by using primers for CBFA-1, ALP, and collagen type I (Li et al., Artif. Organs 2009, 34, 46-54). Glyceraldehide-3-phosphate dehydrogenase (GAPDH) was used as a control for assessing PCR efficiency (Li et al., 2009). The PCR products were size fractionated onto a 2% agarose gel and stained with ethidium bromide. Primers sets and PCR products sizes are listed in Table 2.

**Table 2. Primers sets and PCR products sizes.**

| Gene names | Primers sequences | Product size | Annealing temperature |
|---|---|---|---|
| CBFA-1 (Runx2) | F: 5'CAGACCAGCAGCACTCCATA-3' (SEQ ID NO: 1) | 256 | 52 |
| | R :5'-TCCAATATGGTCGCCAAACA-3' (SEQ ID NO: 2) | | |
| ALP | F: 5'-CCCAAAGGCTTCTTCTTG-3' (SEQ ID NO:3) | 356 | 55 |
| | R: 5-CTGGTAGTTGTTGTGAGCAT-3' (SEQ ID NO: 4) | | |
| Collagen type I | F: 5'-CGCCATCAAGGTCTACTGC-3' (SEQ ID NO: 5) | 148 | 54 |
| | R: 5'-GAATCCATCGGTCATGCTCT-3' (SEQ ID NO: 6) | | |
| GAPDH | F: 5'-ACCACAGTCCATGCCATCAC-3' (SEQ ID NO: 7) | 452 | 55 |
| | R: 5'-TCCACCACCCTGTTGCTGTA-3' (SEQ ID NO: 8) | | |

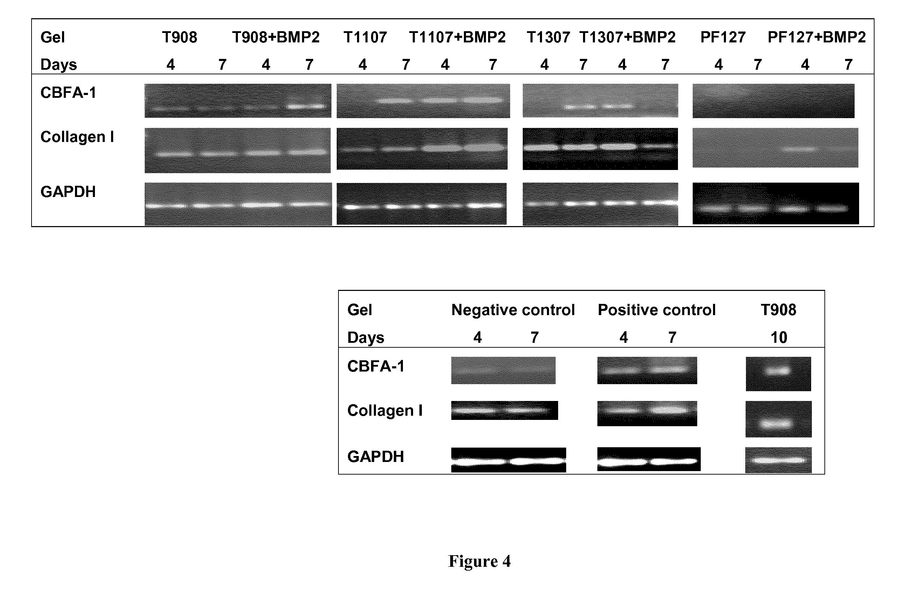

Mesenchymal stem cells exhibited a basal expression of CBFA-1. Tetronic 908, 1107 and 1307 gels with and without rhBMP-2 increased mRNA expression of CBFA-1, in a time dependent manner (Figure 4). In the absence of BMP-2, the expression induced by T1107 and T1307 was evident at day 7, while with T908 the induction was clearly observed latter, at day 10. This finding is in agreement with the high cell proliferation caused by T908. In the presence of BMP-2, the mRNA expression of CBFA-1 occurred faster (at day 4 with T1107 and T1307 and at day 7 with T908 formulations). It is well-known that initial collagen matrix accumulation is essential for sequential expression of the differentiation related proteins, e.g. alkaline phosphatase, bone sialoprotein, and osteocalcin (Hutmacher, D.W., Sunugan, S.L.C., Bone repair and adult stem cells, en: Stem Cells, from Bench to Bedside, Editores Lee, E.H., World Scientific Publishing Co. Singapore, 2004, pág. 451). Negative controls showed a moderate expression of the collagen type I marker, but a significant inductive effect was recorded for T908 with and without BMP-2 (particularly relevant at day 10), and for T1107 with BMP-2 and T1307 both with and without BMP-2 at days 4 and 7. Oppositely, cells cultured with Pluronic F127 did not show induction of these osteogenic marker genes at any time point of the study.

## Claims

1. Use of one or more poloxamines for the manufacturing of a medicinal product.

2. Use of one or more poloxamines for the manufacturing of a medicinal product for inducing the differentiation of mesenchymal or stem cells.

3. Use of one or more poloxamines, according to claim 2, for the manufacturing of a medicinal product for inducing the differentiation of mesenchymal or stem cells into osteoblasts.

4. Use of one or more poloxamines for the manufacturing of a medicinal product for the treatment of bone defects generated by comminutes fractures.

5. Use of one or more poloxamines for the manufacturing of a medicinal product for the treatment of fractures associated to osteopenia.

6. Use of one or more poloxamines for the manufacturing of a medicinal product for the treatment of pseudarthrosis.

7. Use of one or more poloxamines for the manufacturing of a medicinal product for the treatment of the loss of bone stock.

8. One or more poloxamines for use as medicinal product.

9. One or more poloxamines for use in inducing the differentiation of mesenchymal or stem cells.

10. One or more poloxamines, according to claim 9, for use in inducing the differentiation of mesenchymal or stem cells into osteoblasts.

11. One or more poloxamines for use in the treatment of bone defects generated by comminutes fractures.

12. One or more poloxamines for use in the treatment of fractures associated to osteopenia.

13. One or more poloxamines for use in the treatment of pseudarthrosis.

14. One or more poloxamines for use in the treatment of the loss of bone stock.

15. Method for inducing the differentiation of mesenchymal or stem cells comprising administration to a patient in that need thereof a therapeutically effective amount of one or more poloxamines.

16. Method according to claim 15, for inducing the differentiation of mesenchymal or stem cells into osteoblasts comprising administration to a patient in that need thereof a therapeutically effective amount of one or more poloxamines.

17. Method for treatment of bone defects generated by comminutes fractures, the method comprising administering a therapeutically effective amount of one or more poloxamines to a patient in the need of said treatment.

18. Method for treatment of fractures associated to osteopenia, the method comprising administering a therapeutically effective amount of one or more poloxamines to a patient in the need of said treatment.

19. Method for treatment of pseudarthrosis, the method comprising administering a therapeutically effective amount of one or more poloxamines to a patient in the need of said treatment.

20. Method for treatment of the loss of bone stock, the method comprising administering a therapeutically effective amount of one or more poloxamines to a patient in the need of said treatment.

21. Composition pharmaceutically acceptable consisting of one or more poloxamines and one or more excipients.

22. Composition according to claim 20, in the form of solution or aqueous dispersion.

23. Pharmaceutical composition according to any of claims 21 or 22, wherein the total concentration of poloxamines is between 2 and 30%.

24. Pharmaceutical composition according to claim 23, wherein the total concentration of poloxamines is between 10 and 20%.

25. Process for the preparation of a pharmaceutical composition as defined in claims 21 to 24, comprising a) mixing of one or more poloxamines in an aqueous media at pH between 6.5 and 7.5 and at a temperature between 0 and 37 °C, and b) sterilization of the mixture.

26. Process according to claim 25, comprising a further step after step a) and before step b), comprising the addition of an isotonizating agent.

27. Process according to any of claims 25 or 26, wherein the pH is 7.4.

28. Process according to any of claims 25, 26 or 27, wherein the sterilization is carried out by a process selected from the following group: sterile filtration, moist heat sterilization, and ionizing or non-ionizing radiations

29. Method *in vitro* for inducing the differentiation of mesenchymal or stem cells, except human embryonic stem cells, into osteoblasts comprising contacting a) a sterilized solution or aqueous dispersion of one or more poloxamines, with b) mesenchymal or stem cells, except human embryonic stem cells, which are in a culture medium.


SEQUENCE LISTING

<110>  Universidade de Santiago de Compostela

<120>  Use of poloxamines to induce osteogenic differentiation of
       mesenchymal cells

<130>  P6763PC00

<150>  EP11150045
<151>  2011-01-03

<150>  ES P201000670
<151>  2010-05-18

<160>  8

<170>  PatentIn version 3.5

<210>  1
<211>  20
<212>  DNA
<213>  Artificial Sequence

<220>
<223>  Primer F CBFA-1 (Runx2)

<400>  1
cagaccagca gcactccata
20


<210>  2
<211>  20
<212>  DNA
<213>  Artificial Sequence

<220>
<223>  Primer R CBFA-1 (Runx2)

<400>  2
tccaatatgg tcgccaaaca
20


<210>  3
<211>  18
<212>  DNA
<213>  Artificial Sequence

<220>
<223>  Primer F ALP

<400>  3
cccaaaggct tcttcttg
18

<210>  4
<211>  20
<212>  DNA
<213>  Artificial Sequence

<220>
<223>  Primer R ALP

<400>  4
ctggtagttg ttgtgagcat
20



<210>  5
<211>  19
<212>  DNA
<213>  Artificial Sequence

<220>
<223>  Primer F Collagen type I

<400>  5
cgccatcaag gtctactgc
19



<210>  6
<211>  20
<212>  DNA
<213>  Artificial Sequence

<220>
<223>  Primer R Collagen type I

<400>  6
gaatccatcg gtcatgctct
20



<210>  7
<211>  20
<212>  DNA
<213>  Artificial Sequence

<220>
<223>  Primer F GAPDH

<400>  7
accacagtcc atgccatcac
20



<210>  8
<211>  20
<212>  DNA
<213>  Artificial Sequence

<220>
<223>  Primer R GAPDH

<400>  8
tccaccaccc tgttgctgta
20